# EUROPEAN PATENT SPECIFICATION

(11) **EP 3 793 952 B1**
(45) Date of publication and mention of the grant of the patent: **29.05.2024**
(21) Application number: 18918996.2
(22) Date of filing: 14.07.2018
(51) Int. Cl.: C02F 11/04, C12M 1/02, C12M 1/107, C02F 1/66

(54) **PLUG FLOW BIOGAS PLANT**
PFROPFENSTRÖMUNGSBIOGASANLAGE
INSTALLATION DE BIOGAZ À ÉCOULEMENT PISTON

(30) Priority: 17.05.2018 IN 201821018569
(43) Date of publication of application: 24.03.2021
(73) Proprietor: Enel Viento India Private Limited, Balewadi Pune, Maharashtra 411045 (IN)
(72) Inventor: SCHERZ, Narayan, Kullu Himachal Pradesh 175101 (IN)
(74) Representative: Dehns
(86) International application number: PCT/IB2018/055223
(87) International publication number: WO 2019/220191

(56) References cited:
- US-A1- 2005 026 262
- US-A1- 2016 298 067
- US-B2- 8 329 455
- US-B2- 9 409 806
- US-B2- 9 815 039
- JAGADISH KS et al.: "Plug flow digestors for biogas generation from leaf biomass", BIOMASS AND BIOENERGY, vol. 14, no. 5-6, 1 May 1998 (1998-05-01), pages 415-423, XP055653364,

## Description

### TECHNICAL FIELD

The present invention relates generally to biogas plants, and more particularly to a biogas plant that converts biodegradable waste into biogas using anaerobic digestion.

### BACKGROUND OF THE INVENTION

Background description includes information that may be useful in understanding the present invention. It is not an admission that any of the information provided herein is prior art or relevant to the presently claimed invention, or that any publication specifically or implicitly referenced is prior art.

A biogas plant is an anaerobic digester that treats agricultural wastes to generate a mixture of gases, generally termed as biogas, that may be used as a fuel. The gases can be produced using anaerobic digesters that include air-tight tanks having different configurations. These biogas plants can be fed with crops such as maize silage or biodegradable wastes including sewage sludge and food waste. During the digesting process, micro-organisms transform biomass waste into biogas, primarily methane and carbon dioxide.

Various designs of conventional digesters exist for processing and treatment of organic wastes to produce non-hazardous, and sometimes beneficial, products for release to the environment. Digesters may be designed for use in low technology rural areas or for sophisticated industrial areas. A major objective of digestion process carried out in a digester is to reduce the total amount of sludge solids, and to produce a cleaner effluent for discharge to the environment or for further processing prior to discharge. Successful anaerobic digestion of organic wastes usually requires a mixed culture of bacteria with a complex interdependency, terminating in the production of methane by methanogenic bacteria.

A major disadvantage of anaerobic digesters is the long residence time typically required to digest the organic waste. Many anaerobic digesters are one-stage digesters that comprise a closed or domed vessel within which very large quantities of organic waste are fermented in batch. Anaerobic batch digesters can take 20 to 30 days to adequately digest the organic solids. Although these batch digesters can handle large quantities of waste, the prolonged time usually required for digestion has limited their use for municipal or industrial waste.

Furthermore, some limitations associated with anaerobic digesters are the rate at which waste can be processed, and the fraction of solids in the waste that can be digested. The residence time required by standard-rate anaerobic digesters whose contents are unmixed and unheated for the microorganisms to produce a clean effluent is quite long. Optimum anaerobic performance is achieved by proper mixing and heating. Mixing is achieved by gas injection, mechanical stirring, and mechanical pumping. In addition, conventional digesters contain only one chamber that is used for both the hydrolysis process and the agitation process. This tends to reduce efficacy of the digestion process.
U.S. Patent No. 9815039 B2 describes a plug flow bioreactor, which has a long-shaft agitator for thoroughly mixing a biomass with a high solids content.
U.S. Patent Application Publication No. 20160298067 A1 describes a method for producing biogas from biomass using anaerobic digestion using a reactor.
U.S. Patent Application Publication No. 20050026262 A1 describes system and method for sonication-enhanced digestion of cellular matter.
U.S. Patent No. 9409806 B2 describes system and method for thermophilic anaerobic digester process.

There is therefore a need in the art for a portable biogas plant that converts organic waste into sustainable energy by producing biogas that may be used as a fuel. The biogas plant incorporates a digesting unit that allows plug flow of the organic waste and further ensures complete and even mixing of the organic waste.

### OBJECTS OF THE INVENTION

It is an object of the present invention to provide a biogas plant that converts biodegradable waste into biogas using anaerobic digestion.

It is another object of the present invention to provide a biogas plant that effects complete and even mixing of organic waste fed into the biogas plant.

It is another object of the present invention to provide a biogas plant that maintains a uniform quality of the biogas generated thereof.

It is another object of the present invention to provide a biogas plant that envisages complete conversion of the organic matter into the biogas in a defined retention period.

It is another object of the present invention to provide a biogas plant that produces low sludge yields.

It is yet another object of the present invention to provide a biogas plant that envisages increased resistance towards formation of toxic material.

It is still another object of the present invention to provide a biogas plant that offers easy maintenance of pH value of the organic waste.

### SUMMARY

The present invention relates to a biogas generation apparatus as defined in claim 1 that converts biodegradable waste into biogas using anaerobic digestion. The biogas generation apparatus includes a digesting unit to effect hydrolysis and anaerobic digestion of an organic slurry, wherein the digesting unit receives the organic slurry from a feeding unit, a flexible dome located at top of the digesting unit, said flexible dome provided with a plurality of pipelines to convey the biogas from within the digesting unit to a gas pressurization unit to maintain pressure of the biogas stored in a pressurized vessel, and an agitator assembly positioned within the digesting unit, said agitator assembly comprising a shaft coupled with a plurality of arms such that rotation of the agitator assembly about a central axis of the shaft creates plug flow of the organic slurry in the digesting unit.

The digesting unit is separated into a plurality of chambers, at least a hydrolysis chamber of which is adapted for hydrolysis of an organic slurry and at least a digesting chamber of which is adapted for anaerobic digestion of the organic slurry, and wherein rotation of the agitator assembly about the central axis of the shaft separates each of the plurality of chambers from one another.

In an embodiment, the digesting unit is provided with a set of heating elements to maintain pre-defined temperatures in each of the plurality of chambers of the digesting unit.

The biogas generation apparatus further includes a spraying unit comprising a plurality of nozzles, which are in an embodiment adapted to spray the micro-organism into the digesting chamber, and wherein the micro-organism consumes monomers of the organic slurry during anaerobic digestion in the digesting chamber and releases biogas as a byproduct that gets collected in the flexible dome.

A plurality of sensors is configured with each of the plurality of chambers of the digesting unit to detect one or more characteristics of any or a combination of the organic slurry and the apparatus. According to the invention, a plurality of pH sensors are provided to the digesting unit to detect pH value of the slurry passing through different sections of the digesting unit. The biogas generation apparatus further includes a control unit as described in claim 1 and configured to monitor and regulate one or more operational parameters of the apparatus based on said detection of the one or more characteristics of any or a combination of the organic slurry and the apparatus.

The biogas generation apparatus further includes a manure separation unit to separate solid particles from digested slurry passing through the digesting chamber of the digesting unit to obtain a basic fluid that is free of solid particles.

In case there is a change in pH level of the organic matter in any one of the plurality of chambers, the basic fluid is sprayed into the digesting unit to compensate for the change in pH level of the organic matter.

In an embodiment, the feeding unit comprises a crusher pump to crush a biodegradable material as to obtain the organic slurry.

In an embodiment, the flexible dome is coupled with a set of gas flow meters to measure discharge of the biogas passing through the plurality of pipelines.

In an embodiment, the biogas generation apparatus further includes a gas flare unit for burning excess biogas stored in the pressurized vessel.

Those skilled in the art will further appreciate the advantages and superior features of the disclosure together with other important aspects thereof on reading the detailed description that follows in conjunction with the drawings.

### BRIEF DESCRIPTION OF THE DRAWINGS

The accompanying drawings are included to provide a better understanding of the present invention, and are incorporated in and constitute a part of this specification. The drawings illustrate exemplary embodiments of the present invention and, together with the description, serve to explain the principles of the present invention.

In the figures, similar components and/or features may have the same reference label. Further, various components of the same type may be distinguished by following the reference label with a second label that distinguishes among the similar components.
FIG. 1 illustrates an exemplary representation of proposed biogas plant in accordance with an embodiment of the present invention.
FIG. 2 illustrates an exemplary representation of digesting unit of the proposed biogas plant in accordance with an embodiment of the present invention.
FIG. 3 illustrates an exemplary representation of a feeding unit that feeds organic slurry into the digesting unit of the proposed biogas plant in accordance with an embodiment of the present invention.
FIG. 4 illustrates an exemplary representation of agitator assembly positioned in the digesting unit of the proposed biogas plant in accordance with an embodiment of the present invention.
FIG. 5 illustrates an exemplary representation of gas pressurization unit of the proposed biogas plant in accordance with an embodiment of the present invention.
FIG. 6 illustrates an exemplary representation of gas flare unit of the proposed biogas plant in accordance with an embodiment of the present invention.

### DETAILED DESCRIPTION

The present invention relates to a biogas generation apparatus as described in claim 1 that converts biodegradable waste into biogas using anaerobic digestion. The apparatus includes a digesting unit to effect hydrolysis and anaerobic digestion of an organic slurry, wherein the digesting unit receives the organic slurry from a feeding unit, a flexible dome located at top of the digesting unit, said flexible dome provided with a plurality of pipelines to convey the biogas from within the digesting unit to a gas pressurization unit to maintain pressure of the biogas stored in a pressurized vessel, and an agitator assembly positioned within the digesting unit, said agitator assembly comprising a shaft coupled with a plurality of arms such that rotation of the agitator assembly about a central axis of the shaft creates plug flow of the organic slurry in the digesting unit.

The digesting unit is separated into a plurality of chambers, at least a hydrolysis chamber of which is adapted for hydrolysis of the organic slurry and at least a digesting chamber of which is adapted for anaerobic digestion of the organic slurry, and wherein rotation of the agitator assembly about the central axis of the shaft separates each of the plurality of chambers from one another.

In an embodiment, the digesting unit is provided with a set of heating elements to maintain pre-defined temperatures in each of the plurality of chambers of the digesting unit.

The biogas generation apparatus further includes a spraying unit comprising a plurality of nozzles, which are in an embodiment adapted to spray the micro-organism into the digesting chamber, and wherein the micro-organism consumes monomers of the organic slurry during anaerobic digestion in the digesting chamber and releases biogas as a byproduct that gets collected in the flexible dome.

A plurality of sensors is configured with each of the plurality of chambers of the digesting unit to detect one or more characteristics of any or a combination of the organic slurry and the apparatus. According to the invention, a plurality of pH sensors are provided to the digesting unit to detect pH value of the slurry passing through different sections of the digesting unit. The biogas generation apparatus further includes a control unit as defined in claim 1 and configured to monitor and regulate one or more operational parameters of the apparatus based on said detection of the one or more characteristics of any or a combination of the organic slurry and the apparatus.

The biogas generation apparatus further includes a manure separation unit to separate solid particles from digested slurry passing through the digesting chamber of the digesting unit to obtain a basic fluid that is free of solid particles.

In case there is a change in pH level of the organic matter in any one of the plurality of chambers, the basic fluid is sprayed into the digesting unit to compensate for the change in pH level of the organic matter.

In an embodiment, the feeding unit comprises a crusher pump to crush a biodegradable material as to obtain the organic slurry.

In an embodiment, the flexible dome is coupled with a set of gas flow meters to measure discharge of the biogas passing through the plurality of pipelines.

In an embodiment, the biogas generation apparatus further includes a gas flare unit for burning excess biogas stored in the pressurized vessel.

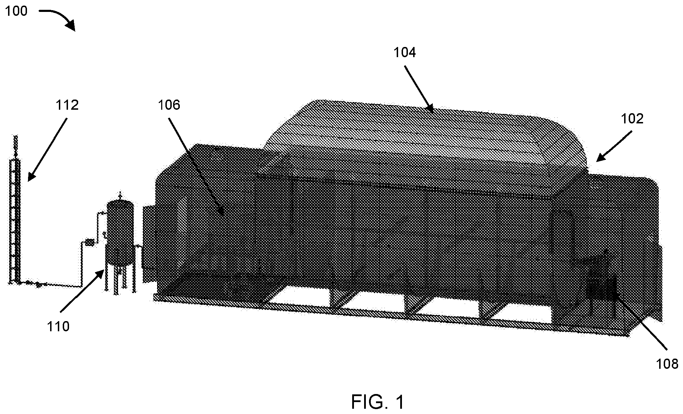

FIG. 1 illustrates an exemplary representation of proposed biogas plant in accordance with an embodiment of the present invention. The proposed biogas plant (interchangeable referred to as biogas generation apparatus) 100 includes a digesting unit 102 having separate chambers for hydrolysis and digestion of the organic slurry, as contrary to conventional biogas plants, to improve even mixing of the organic slurry and to maintain a uniform quality of the biogas generated thereof.

In an embodiment, biodegradable materials such as agricultural waste, crops and the like are fed to a feeding unit 106 and an equal amount of hot water at 40 ⁰C is added thereto. The feeding unit 106 may include a crusher/grinder pump that crushes the biodegradable material to generate the organic slurry containing a suspension of crushed biodegradable material and water. The organic slurry is then fed into a chamber of the digesting unit 102.

The digesting unit 102 includes a plurality of chambers. A hydrolysis chamber of the plurality of chambers is adapted to facilitate hydrolysis of the organic slurry when the organic slurry is fed, from the feeding unit 106, into the hydrolysis chamber. The biodegradable material in the organic slurry contains complex polymers, for example, proteins, lipids and carbohydrates. The hydrolysis process takes place in the hydrolysis chamber and converts the complex polymers into monomers such as but not limited to, simple sugar, amino acids and fatty acid. As the slurry is continuously fed to the digesting unit 102, the slurry enters in a digesting chamber and anaerobic digestion of the slurry tales place.

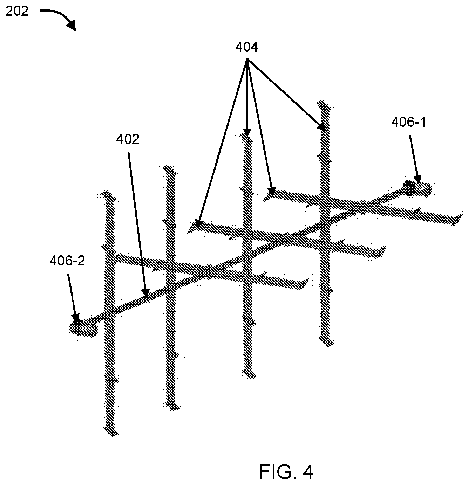

As the digestion process is taking place in absence of oxygen the process is known as anaerobic digestion. During the digestion process, an agitator assembly (as shown in FIG. 4) is continuously rotating within the digestion tank 102 which creates a plug flow of the slurry inside the digestion tank 102 and also separates each of the plurality of chambers of the digesting unit 102 from one another.

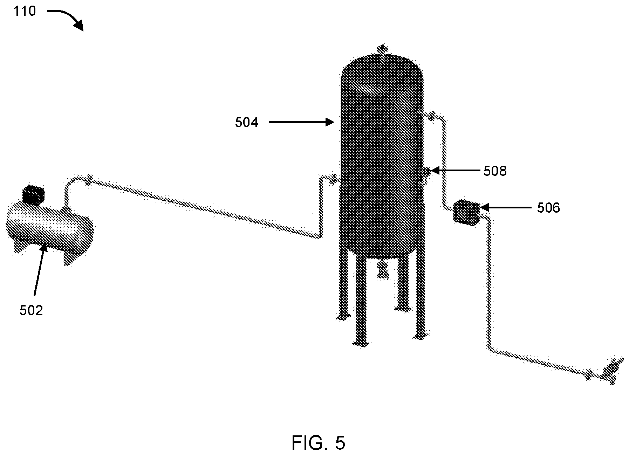

The apparatus 100 includes a flexible dome 104 located at top of the digesting unit 102. After hydrolysis and digestion of the slurry in the primary and the digesting chambers respectively, the biogas generated thereof being lighter than the slurry moves upwards and gets collected in the flexible dome 104. The flexible dome 104 is provided with a plurality of pipelines to convey the biogas collected therein to a gas pressurization unit 110 to maintain pressure of the biogas stored in a pressurized vessel (as shown in FIG. 5). In an embodiment, the biogas stored in the pressurized vessel may be used for various energy generating applications, such as, for generating electricity, for cooling purposes, for heating purposes and the like applications.

In an embodiment, the flexible dome 104 may be coupled with a set of gas flow meters to measure discharge of the biogas passing through the plurality of pipelines. In an embodiment, the apparatus 100 may further include a gas flare unit 112 located at a safe distance from the apparatus, for burning excess biogas stored in the pressurized vessel.

The apparatus 100 further includes a spraying unit (not shown) including a plurality of nozzles, which is in an embodiment adapted to spray the micro-organism into the digesting chamber. The micro-organism consumes monomers of the organic slurry during anaerobic digestion in the digesting chamber and releases biogas as a byproduct that gets collected in a flexible dome 104.

In an embodiment, a plurality of heating elements is configured with the digesting unit 102 to maintain a pre-defined temperature inside the digesting unit 102. In addition, a plurality of sensing devices, such as temperature sensors, pH sensors and the like are configured with each chamber of the digesting unit 102 to detect various characteristics of the organic slurry and the apparatus 100. For example, temperature sensors may be provided with each chamber of the digesting unit 102 to detect temperature of various sections of the digesting unit 102 and the organic slurry passing through each chamber of the digesting unit 102. According to the invention, a plurality of pH sensors are provided to the digesting unit 102 to detect pH value of the slurry passing through different sections of the digesting unit 102.

The apparatus 100 further includes a control unit (not shown) configured to monitor as well as control various operational parameters of the apparatus 100 based on detection of the characteristics of any or a combination of the slurry and the apparatus 100 by the plurality of sensors. For instance, in case the temperature sensors detect a gradual decrease in temperature within the digesting unit 102, the control unit may activate the heating elements to effectively increase temperature within the digesting unit 102 up to a pre-defined temperature. In an embodiment, the control unit may include a display screen on which the detected characteristics of any or a combination of the slurry and the apparatus 100 may be displayed.

In an embodiment, the control unit may be a flat, often vertical, box-shaped device where control or monitoring instruments are displayed or it may be an enclosed unit that is the part of a system that users can access. Using the control unit a user can easily control operational parameters of the apparatus 100, for example, the user can start or stop an individual unit of the apparatus 100. The control unit may also include certain automatic switches that get in operation with the help of the sensors.

The apparatus 100 further includes a manure separation unit 108 to separate solid particles from digested slurry passing through the digesting chamber of the digesting unit 102 to obtain a basic fluid that is free of solid particles. In case there is a change in pH level of the organic matter in any one of the plurality of chambers, the control unit actuates the spraying unit and enables spraying of the basic fluid into the digesting unit to compensate for the change in pH level of the organic slurry.

In an embodiment, the digested slurry from the digesting unit 102 may enter the manure separation unit 108 through a pipe connected to the digesting chamber. The manure separator can separate solid particles from the slurry resulting in the moisture content in the solid manure less than, say 30 %, thereby allowing separation of the solid particles and basic fluid waste that may be used as a bio-fertilizer due to its high value of Nitrogen, Phosphorus, Potassium (NPK). A portion of basic fluid, for instance, 20% may be circulated back into the digesting unit 102 to maintain pH value and also to enhance the digestion process.

In an embodiment, the proposed biogas generation apparatus is modular in nature with all its components being interconnected as to perform its intended function. Furthermore, the proposed biogas generation apparatus may be portable in a way that all its components can be transported from one place to another with ease, and can be interconnected so as to perform intended function of the biogas generation apparatus.

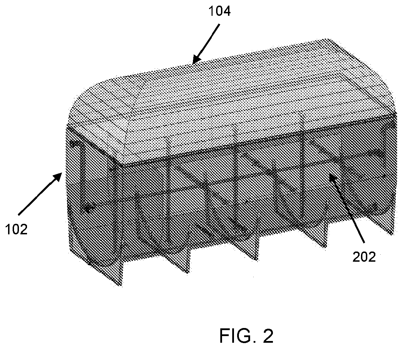

FIG. 2 illustrates an exemplary representation of the digesting unit of the proposed biogas plant in accordance with an embodiment of the present invention. In an aspect, the digesting unit 102 includes a plurality of chambers, namely, a hydrolysis chamber to facilitate hydrolysis of the organic slurry and a digesting chamber to facilitate anaerobic digestion of the organic slurry. The biodegradable material in the organic slurry contains complex polymers, for example, proteins, lipids and carbohydrates. The hydrolysis process takes place in the hydrolysis chamber and converts the complex polymers into monomers such as but not limited to, simple sugar, amino acids and fatty acid. As the slurry is continuously fed to the digesting unit, the slurry enters in a digesting chamber and anaerobic digestion of the slurry tales place. The agitator assembly 202 is positioned within the digesting unit 102 such that the agitator assembly 202 is aligned with longitudinal direction of the digesting unit 102. Rotation of the agitation assembly 202 can allow separation of the plurality of chambers of the digesting unit 102 by inhibiting communication of particles of organic slurry present in adjacent chambers of the digesting unit 102.

The flexible dome 104 is located at top of the digesting unit 102. After hydrolysis and digestion of the slurry in the primary and the digesting chambers respectively, the biogas generated thereof can move upwards and can get collected in the flexible dome 104.

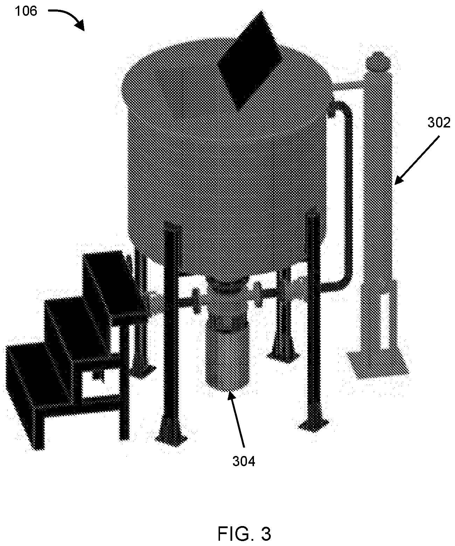

Referring to FIG. 3, where an exemplary representation of the feeding unit 106 that feeds organic slurry into the digesting unit 102 of the proposed biogas plant 100 is shown, biodegradable materials such as agricultural waste, crops and the like are fed to the feeding unit 106 and an equal amount of hot water, heated by a water heater 302, is added thereto. The feeding unit 106 may include a crusher pump 304 that crushes the biodegradable material to generate the organic slurry containing a suspension of crushed biodegradable material and water. The organic slurry is then fed into the hydrolysis chamber of the digesting unit 102.

Referring to FIG. 4, where an exemplary representation of the agitator assembly 202 positioned in the digesting unit 102 of the proposed biogas 100 plant is shown, the agitator assembly 202 includes a shaft 402 coupled with a plurality of arms 404 such that rotation of the agitator assembly 202 about a central axis of the shaft 402 creates plug flow of the organic slurry in the digesting unit, where velocity of the slurry at different locations of the digesting unit 102 is same at any instant. In an embodiment, the agitator assembly 202 is positioned within the digesting unit 102 and is in alignment with longitudinal direction of the digesting unit 102. In an embodiment, rotation of the agitator assembly 202 about the central axis of the shaft 402 separates each of the plurality of chambers from one another and further creates plug flow of the organic slurry in the digesting unit 102. In an embodiment, ends of the shaft 402 is coupled with rotary devices, such as, but not limited to, motors 406-1/406-2 to enable rotation of the shaft 402.

FIG. 5 illustrates an exemplary representation of gas pressurization unit 110 of the proposed biogas plant 100 in accordance with an embodiment of the present invention. The plurality of pipelines coupled with the flexible dome 104 facilitates transfer of the biogas generated by the proposed biogas plant 100 from within the digesting unit 102 to the gas pressurization unit 110 to maintain pressure of the biogas stored in a pressurized vessel 504. The gas pressurization unit 110 may include a gas compressor 502 that compresses the biogas under pressure suitable for effectively supplying the biogas from the pressurized vessel 504 to energy generating devices, such as generators, stoves, heaters and the likes. In an exemplary embodiment, the pressurized vessel 504 is further coupled to a flow meter 506 which is configured to measure the discharge of gas. In an exemplary embodiment, the pressurized vessel 504 can be connected with one or more pressure sensors 508 to detect pressure parameters. For example, the pressure level rises above the maximum permissible pressure or the below minimum permissible pressure is detected by said pressure sensors 508.

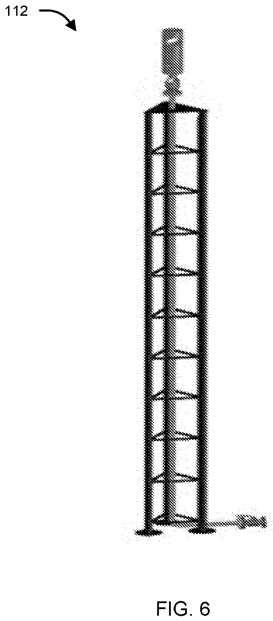

Referring now to FIG. 6, where an exemplary representation of gas flare unit 112 of the proposed biogas plant is shown, the gas flare unit 112 may be adapted for burning excess biogas stored in the pressurized vessel 504 that are not required to pass to the energy generating devices. The gas flare unit 112 is provided at a safe distance from the proposed biogas plant 100 and can burn the excess biogas stored in the pressurized vessel 504 by combusting the excess biogas. The gas flare unit 112 may be automatically actuated by the control unit based on a set of pressure sensors configured within the pressurized vessel 504 to detect pressure parameters, for example, if the pressure level rises above the maximum permissible pressure in 504 automatically actuates the flare unit 112.

Thus, the present invention provides a biogas generation apparatus as defined in claim 1 that includes a digesting unit to effect hydrolysis and anaerobic digestion of the organic slurry, wherein the digesting unit receives the organic slurry from a feeding unit, a flexible dome located at top of the digesting unit, said flexible dome provided with a plurality of pipelines to convey the biogas from within the digesting unit to a gas pressurization unit to maintain pressure of the biogas stored in a pressurized vessel, and an agitator assembly positioned within the digesting unit, said agitator assembly comprising a shaft coupled with a plurality of arms such that rotation of the agitator assembly about a central axis of the shaft creates plug flow of the organic slurry in the digesting unit. The digesting unit is separated into a plurality of chambers, at least a hydrolysis chamber of which is adapted for hydrolysis of the organic slurry and at least a digesting chamber of which is adapted for anaerobic digestion of the organic slurry, and wherein rotation of the agitator assembly about the central axis of the shaft separates each of the plurality of chambers from one another. The apparatus is further defined in claim 1. Preferred embodiments are described in the dependent claims 2-7.

### ADVANTAGES OF THE INVENTION

The present invention provides a biogas plant that converts biodegradable waste into biogas using anaerobic digestion.

The present invention provides a biogas plant that effects complete and even mixing of organic waste fed into the biogas plant.

The present invention provides a biogas plant that maintains a uniform quality of the biogas generated thereof.

The present invention e provides a biogas plant that envisages complete conversion of the organic matter into the biogas in a defined retention period.

The present invention provides a biogas plant that produces low sludge yields.

The present invention provides a biogas plant that envisages increased resistance towards formation of toxic material.

The present invention provides a biogas plant that offers easy maintenance of pH value of the organic waste.

The present disclosure provides a biogas plant that envisages complete conversion of the organic matter into the biogas in a defined retention period.

The present disclosure provides a biogas plant that produces low sludge yields.

The present disclosure provides a biogas plant that envisages increased resistance towards formation of toxic material.

The present disclosure provides a biogas plant that offers easy maintenance of pH value of the organic waste.

## Claims

1. A biogas generation apparatus comprising:
a feeding unit (106);
a digesting unit (102) to effect hydrolysis and anaerobic digestion of an organic slurry, wherein the digesting unit (102) is arranged to receive the organic slurry from said feeding unit (106);
a gas pressurization unit (110);
a flexible dome (104) located at top of the digesting unit (102), said flexible dome (104) provided with a plurality of pipelines to convey the biogas from within the digesting unit (102) to the gas pressurization unit (110) to maintain pressure of the biogas stored in a pressurized vessel;
an agitator assembly (202) positioned within the digesting unit (102), said agitator assembly (202) comprising a shaft coupled with a plurality of arms such that rotation of the agitator assembly (202) about a central axis of the shaft creates plug flow of the organic slurry in the digesting unit (102);
wherein the digesting unit (102) is separated into a plurality of chambers, at least a hydrolysis chamber of which is adapted for hydrolysis of an organic slurry and at least a digesting chamber of which is adapted for anaerobic digestion of the organic slurry, and wherein rotation of the agitator assembly (202) about the central axis of the shaft separates each of the plurality of chambers from one another;
a manure separation unit (108) connected to the digesting unit (102) to separate solid particles from digested slurry that has passed through the digesting unit (102) to obtain a basic fluid that is free of solid particles;
a plurality of sensors comprising pH sensors provided to the digesting unit (102) to detect pH value of the slurry passing through different sections of the digesting unit (102);
a spraying unit comprising a plurality of nozzles; and
a control unit configured to actuate the spraying unit and enable spraying of the basic fluid into the digesting unit (102) to compensate for the change in the pH level of the organic slurry, in case there is a change in pH level of the organic matter in any one of the plurality of chambers.

2. The biogas generation apparatus as claimed in claim 1, wherein the digesting unit (102) is provided with a set of heating elements to maintain pre-defined temperatures in each of the plurality of chambers of the digesting unit (102).

3. The biogas generation apparatus as claimed in claim 1, wherein the plurality of nozzles are adapted to spray the micro-organism into the digesting chamber, and wherein the micro-organism consumes monomers of the organic slurry during anaerobic digestion in the digesting chamber and releases biogas as a byproduct that gets collected in the flexible dome.

4. The biogas generation apparatus as claimed in claim 1, wherein the feeding unit (106) comprises a crusher pump to crush a biodegradable material as to obtain the organic slurry.

5. The biogas generation apparatus as claimed in claim 1, wherein the flexible dome (104) is coupled with a set of gas flow meters to measure discharge of the biogas passing through the plurality of pipelines.

6. The biogas generation apparatus as claimed in claim 1, wherein said gas pressurization unit (110) further comprises a biogas compressor (502) adapted to raise a pressure of the gas so as to make the flow of the gas more fluently.

7. The biogas generation apparatus as claimed in claim 1, further comprising a gas flare unit (112) adapted to burn excess biogas stored in the pressurized vessel.

## Patentansprüche

1. Biogaserzeugungseinrichtung, umfassend:
eine Zufuhreinheit (106);
eine Vergärungseinheit (102), um eine Hydrolyse und eine anaerobe Vergärung eines organischen Schlamms herbeizuführen, wobei die Vergärungseinheit (102) angeordnet ist, um den organischen Schlamm von der Zufuhreinheit (106) aufzunehmen;
eine Gasdruckaufbaueinheit (110);
eine flexible Kuppel (104), die sich oben auf der Vergärungseinheit (102) befindet, wobei die flexible Kuppel (104) mit einer Vielzahl von Rohrleitungen bereitgestellt ist, um das Biogas aus dem Inneren der Vergärungseinheit (102) zur Gasdruckaufbaueinheit (110) zu befördern, um den Druck des in einem druckbeaufschlagten Gefäß gespeicherten Biogases aufrechtzuerhalten;
eine Rühranordnung (202), die im Inneren der Vergärungseinheit (102) positioniert ist, wobei die Rühranordnung (202) eine Welle umfasst, die mit einer Vielzahl von Armen gekoppelt ist, so dass eine Drehung der Rühranordnung (202) um eine Mittelachse der Welle eine Pfropfenströmung des organischen Schlamms in der Vergärungseinheit (102) erzeugt;
wobei die Vergärungseinheit (102) in eine Vielzahl von Kammern unterteilt ist, von denen mindestens eine Hydrolysekammer für die Hydrolyse eines organischen Schlamms angepasst ist und von denen mindestens eine Vergärungskammer für die anaerobe Vergärung des organischen Schlamms angepasst ist, und wobei eine Drehung der Rühranordnung (202) um die Mittelachse der Welle jede der Vielzahl von Kammern voneinander trennt;
eine Gülle-Abscheideeinheit (108), die mit der Vergärungseinheit (102) verbunden ist, um Feststoffpartikel aus dem vergorenen Schlamm abzuscheiden, der durch die Vergärungseinheit (102) durchgelaufen ist, um eine Basisflüssigkeit zu erhalten, die frei von Feststoffpartikeln ist;
eine Vielzahl von Sensoren, die pH-Sensoren umfassen, die an der Vergärungseinheit (102) bereitgestellt sind, um einen pH-Wert des Schlamms zu erfassen, der verschiedene Abschnitte der Vergärungseinheit (102) durchlaufen hat;
eine Sprüheinheit, die eine Vielzahl von Düsen umfasst; und
eine Steuereinheit, die konfiguriert ist, um die Sprüheinheit zu betätigen und ein Sprühen der Basisflüssigkeit in die Vergärungseinheit (102) zu ermöglichen, um die Änderung des pH-Werts des organischen Schlamms auszugleichen, falls es zu einer Änderung des pH-Werts der organischen Substanz in einer der Vielzahl von Kammern kommt.

2. Biogaserzeugungseinrichtung nach Anspruch 1, wobei die Vergärungseinheit (102) mit einer Reihe von Heizelementen bereitgestellt ist, um in jeder der Vielzahl von Kammern der Vergärungseinheit (102) vordefinierte Temperaturen aufrechtzuerhalten.

3. Biogaserzeugungseinrichtung nach Anspruch 1, wobei die Vielzahl von Düsen angepasst ist, um den Mikroorganismus in die Vergärungskammer zu sprühen, und wobei der Mikroorganismus während einer anaeroben Vergärung in der Vergärungskammer Monomere des organischen Schlamms verbraucht, und Biogas als Nebenprodukt freisetzt, das in der flexiblen Kuppel gesammelt wird.

4. Biogaserzeugungseinrichtung nach Anspruch 1, wobei die Zufuhreinheit (106) eine Zerkleinerungspumpe umfasst, um ein biologisch abbaubar Material zu zerkleinern, um den organischen Klärschlamm zu erhalten.

5. Biogaserzeugungseinrichtung nach Anspruch 1, wobei die flexible Kuppel (104) mit einer Reihe von Gasdurchflussmessern gekoppelt ist, um den Ausstoß des durch die Vielzahl von Rohrleitungen durchlaufenden Biogases zu messen.

6. Biogaserzeugungseinrichtung nach Anspruch 1, wobei die Gasdruckaufbaueinheit (110) weiter einen Biogaskompressor (502) umfasst, der angepasst ist, um den Druck des Gases zu erhöhen, um den Gasfluss flüssiger zu gestalten.

7. Biogaserzeugungseinrichtung nach Anspruch 1, die weiter eine Gasfackeleinheit (112) umfasst, die angepasst ist, um im druckbeaufschlagten Gefäß gespeichertes überschüssiges Biogas zu verbrennen.

## Revendications

1. Appareil de génération de biogaz comprenant :
une unité d'alimentation (106) ;
une unité de digestion (102) pour effectuer l'hydrolyse et la digestion anaérobie d'une suspension organique, dans lequel l'unité de digestion (102) est conçue pour recevoir la suspension organique provenant de ladite unité d'alimentation (106) ;
une unité de pressurisation de gaz (110) ;
un dôme flexible (104) situé au sommet de l'unité de digestion (102), ledit dôme flexible (104) étant doté d'une pluralité de canalisations pour transporter le biogaz de l'intérieur de l'unité de digestion (102) à l'unité de pressurisation de gaz (110) pour maintenir la pression du biogaz stocké dans une cuve sous pression ;
un ensemble agitateur (202) positionné à l'intérieur de l'unité de digestion (102), ledit ensemble agitateur (202) comprenant un arbre couplé à une pluralité de bras de telle sorte que la rotation de l'ensemble agitateur (202) autour d'un axe central de l'arbre crée un écoulement piston de la suspension organique dans l'unité de digestion (102) ;
dans lequel l'unité de digestion (102) est séparée en une pluralité de chambres, dont au moins une chambre d'hydrolyse est conçue pour l'hydrolyse d'une suspension organique et dont au moins une chambre de digestion est conçue pour une digestion anaérobie de la suspension organique, et dans lequel la rotation de l'ensemble agitateur (202) autour de l'axe central de l'arbre sépare chacune de la pluralité de chambres les unes des autres ;
une unité de séparation de fumier (108) reliée à l'unité de digestion (102) pour séparer les particules solides de la suspension digérée qui est passée à travers l'unité de digestion (102) pour obtenir un fluide basique exempt de particules solides ;
une pluralité de capteurs comprenant des capteurs de pH disposés sur l'unité de digestion (102) pour détecter la valeur du pH de la suspension passant à travers différentes sections de l'unité de digestion (102) ;
une unité de pulvérisation comprenant une pluralité de buses ; et
une unité de commande configurée pour actionner l'unité de pulvérisation et permettre la pulvérisation du fluide basique dans l'unité de digestion (102) pour compenser le changement du niveau de pH de la suspension organique, en cas de changement du niveau de pH de la matière organique dans l'une quelconque de la pluralité de chambres.

2. Appareil de génération de biogaz selon la revendication 1, dans lequel l'unité de digestion (102) est dotée d'un ensemble d'éléments chauffants pour maintenir des températures prédéfinies dans chacune de la pluralité de chambres de l'unité de digestion (102).

3. Appareil de génération de biogaz selon la revendication 1, dans lequel la pluralité de buses sont conçues pour pulvériser le micro-organisme dans la chambre de digestion, et dans lequel le micro-organisme consomme des monomères de la suspension organique pendant la digestion anaérobie dans la chambre de digestion et libère du biogaz comme sous-produit collecté dans le dôme flexible.

4. Appareil de génération de biogaz selon la revendication 1, dans lequel l'unité d'alimentation (106) comprend une pompe broyeuse pour broyer un matériau biodégradable de manière à obtenir la suspension organique.

5. Appareil de génération de biogaz selon la revendication 1, dans lequel le dôme flexible (104) est couplé à un ensemble de débitmètres de gaz pour mesurer la sortie du biogaz passant à travers la pluralité de canalisations.

6. Appareil de génération de biogaz selon la revendication 1, dans lequel ladite unité de pressurisation de gaz (110) comprend en outre un compresseur de biogaz (502) conçu pour accroître la pression du gaz de manière à rendre l'écoulement du gaz plus fluide.

7. Appareil de génération de biogaz selon la revendication 1, comprenant en outre une unité de torchage de gaz (112) conçue pour brûler l'excès de biogaz stocké dans la cuve sous pression.
